# EUROPEAN PATENT APPLICATION

(11) **EP 4 595 774 A1**
(43) Date of publication of application: **06.08.2025**
(21) Application number: 23870812.7
(22) Date of filing: 26.09.2023
(51) Int. Cl.: A23L 33/10, A23L 33/12, A61K 31/19, A61P 3/04

(54) **COMPLEX OF 3-HYDROXYBUTYRIC ACID AND SODIUM 3-HYDROXYBUTYRATE AND PREPARATION METHOD THEREFOR**

(30) Priority: 27.09.2022 WO PCT/CN2022/121659
(71) Applicant: Nanjing Nutrabuilding Bio-Tech Co., Ltd., Nanjing, Jiangsu 210017 (CN)
(72) Inventor: RONG, Liang, Nanjing, Jiangsu 210017 (CN); ZHU, Jinjian, Nanjing, Jiangsu 210017 (CN); CHEN, Youjian, Nanjing, Jiangsu 210017 (CN); JIANG, Long, Nanjing, Jiangsu 210017 (CN); ZHU, Xi, Nanjing, Jiangsu 210017 (CN); LIAO, Kylin, Nanjing, Jiangsu 210017 (CN); WELLS, Shawn, Nanjing, Jiangsu 210017 (CN)
(74) Representative: Frick, Robert
(86) International application number: PCT/CN2023/121529
(87) International publication number: WO 2024/067584

(57) **Abstract**

The invention discloses a complex of 3-hydroxybutyric acid and sodium 3-hydroxybutyrate and a preparation method thereof. The complex provided by the invention effectively solves the acid/salt load and gastrointestinal side effects associated with the existing products, has good taste comfort, and has a good ketogenic effect, and can be better used as a dietary or nutritional supplement.

## Description

### FIELD OF THE INVENTION

This invention belongs to the technical field of dietary or nutritional supplements, specifically relates to a complex of 3-hydroxybutyric acid and sodium 3-hydroxybutyrate and a preparation method therefor.

### BACKGROUND OF THE INVENTION

At present, people's demand for dietary or nutritional supplements is increasing, aiming at improving individual health and reducing disease risk. Athletes also use dietary or nutritional supplements to improve their strength and performance.

While normally human body would rely on glucose for energy, when the supply of glucose is too low for the body's energy needs, such as during periods of prolonged exercise, starvation, or absence of dietary carbohydrates, the body will turn to consume fat as fuel. Since the brain and central nervous system cannot directly use fat for energy, the liver produces ketone bodies (also known as ketones) from fatty acids as an alternative fuel source, which are then released into the blood/plasma. Ketones not only provide fuel for the brain, but are also used by the skeletal and heart muscle. The metabolism of ketone bodies is associated with several beneficial effects, including anticonvulsant effects, enhanced brain metabolism, neuroprotection, muscle sparing properties, and improved cognitive and physical performance. Science-based improvements in efficiency of cellular metabolism, managed through ketone supplementation, can have beneficial impacts on physical, cognitive health, and psychological health, and a long-term impact on health with respect to common avoidable diseases such as obesity, cardiovascular disease, neurodegenerative diseases, diabetes, and cancer.

Despite the many health advantages of pursuing a ketogenic diet or lifestyle and maintaining a state of nutritional ketosis, there remain significant barriers to pursuing and maintaining a ketogenic state. One of these barriers is the difficulty of transitioning into a ketogenic state. The fastest endogenous way to entering ketosis through depleting glucose stores in the body is by fasting combined with exercise. This is physically and emotionally demanding and is extremely challenging even for the most motivated and disciplined.

A large number of studies on exogenous ketones show that the intake of compounds that can increase the level of ketone bodies in blood can bring various clinical benefits, including enhancing physical and cognitive abilities and treating cardiovascular diseases, diabetes, neurodegenerative diseases and epilepsy. Therefore, it is desirable to provide ketone bodies as energy directly to humans or animals. Dietary or nutritional supplements may contain carboxylic acids, such as β-hydroxybutyric acid (also known as 3-hydroxybutyric acid or BHB), which is one of the three main ketone bodies (namely acetoacetate, acetone and BHB).

However, currently known ingestible exogenous ketone bodies have the disadvantage of limiting their use. β-hydroxybutyric acid is a source of exogenous ketones, and its well-known problem is extremely strong acidity. Because of this acidity, the amount and concentration of β-hydroxybutyric acid used in ingestible form are limited. By converting β-hydroxybutyric acid into its sodium, magnesium, calcium and potassium salts, the acidity problem of D-BHB acid has been solved in some applications. However, although salt can solve the problem of acidity, it is easy to lead to electrolyte imbalance due to salt overload, and the use of ketone salt is also limited to a very small amount, which is not only a small dose, but also unpleasant. When BHB acid and salt are simply physically mixed, the above problems still exist, and it is difficult to achieve uniform mixing.

Therefore, in order to solve the problems of strong acidity and high hygroscopicity of the existing BHB acid, high salt load, gastrointestinal side effects and unpleasant taste of the existing BHB salt, it is necessary to further find substances that can effectively avoid or balance the above problems, so as to be better used as ketogenic substances in diet or nutritional supplements, especially for the preparation of solid granules and the application of granules.

### SUMMARY OF THE INVENTION

In one aspect, the present invention provides a complex comprising 3-hydroxybutyric acid and sodium 3-hydroxybutyrate.

In some embodiments, the complex comprises 3-hydroxybutyrate anions, sodium ions and hydrogen ions. In some embodiments, anions in the complex structure include 3-hydroxybutyrate anions, and cations include sodium ions and hydrogen ions.

In some embodiments, the content of 3-hydroxybutyrate anions in the complex is greater than that in sodium 3-hydroxybutyrate.

In some embodiments, the ratio of 3-hydroxybutyric acid to sodium 3-hydroxybutyrate is 1:10 to 10:1. In some embodiments, the ratio of 3-hydroxybutyric acid to sodium 3-hydroxybutyrate can be 1:10 to 10:1, 1:8 to 8:1, 1:5 to 5:1, 1:3 to 3:1, 1:2 to 2:1, 1:1.

In some embodiments, the complex contains not less than 50% of R configuration, not more than 50% of S configuration; or more than 50% of S configuration, less than 50% of R configuration.

In some embodiments, the complex is 3-hydroxybutyric acid · sodium 3-hydroxybutyrate.

In some embodiments, the complex has the following structure:

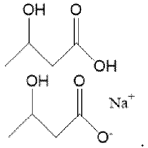

In some embodiments, the complex is R-3-hydroxybutyric acid · sodium R-3-hydroxybutyrate and/or S-3-hydroxybutyric acid · sodium S-3-hydroxybutyrate.

In some embodiments, the complex contains not less than 50% of R-3-hydroxybutyric acid · sodium R-3-hydroxybutyrate, not more than 50% of S-3-hydroxybutyric acid · sodium S-3-hydroxybutyrate; or more than 50% of S-3-hydroxybutyric acid · sodium S-3-hydroxybutyrate, less than 50% of R-3-hydroxybutyric acid · sodium R-3-hydroxybutyrate. In some embodiments, the complex may contain 51-100%, 51-99%, 55-95%, 60-90%, 70-80% of R-3-hydroxybutyric acid · sodium R-3-hydroxybutyrate; 0-49%, 1-49%, 5-45%, 10-40%, 20-30% of S-3-hydroxybutyric acid · sodium S-3-hydroxybutyrate. In some embodiments, the complex may contain 51-100%, 51-99%, 55-95%, 60-90%, 70-80% of S-3-hydroxybutyric acid · sodium S-3-hydroxybutyrate; 0-49%, 1-49%, 5-45%, 10-40%, 20-30% of R-3-hydroxybutyric acid · sodium R-3-hydroxybutyrate.

In some embodiments, the complex has the following structures:

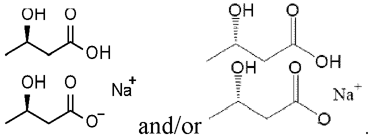

In some embodiments, the complex is in crystalline form.

In some embodiments, the X-ray powder diffraction pattern of the complex comprises peaks at diffraction angles (2θ) of 9.8°±0.2°, 10.8°±0.2°, 18.9±0.2°, and 31.1°±0.2°.

In some embodiments, the X-ray powder diffraction pattern of the complex further comprises one or more peaks at diffraction angles (2θ) of 13.4°±0.2°, 21.6°±0.2°, 32.7°±0.2°.

In some embodiments, the X-ray powder diffraction pattern of the complex further comprises one or more peaks at diffraction angles (2θ) of 15.1°±0.2°, 23.0°±0.2°, 31.9°±0.2°.

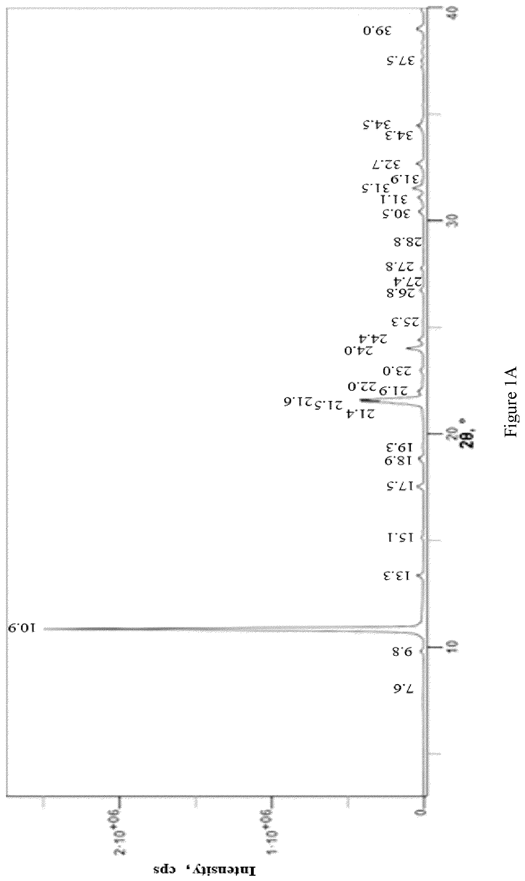

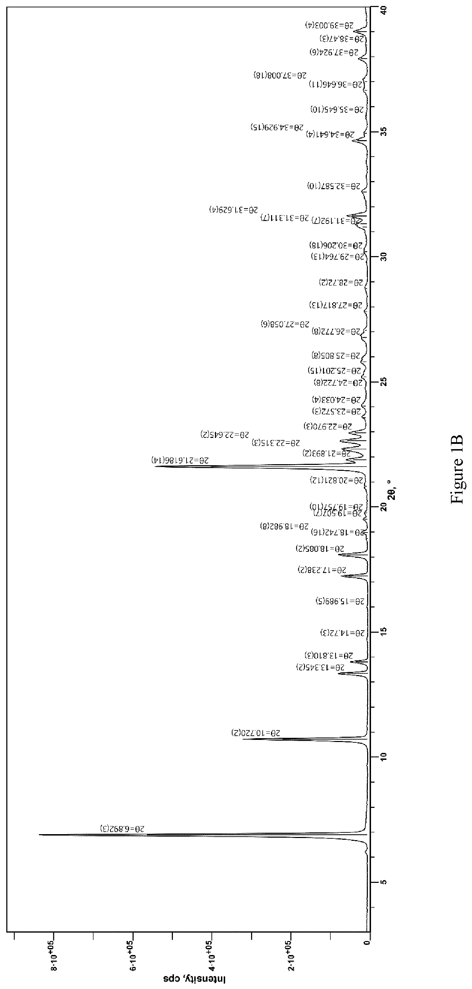

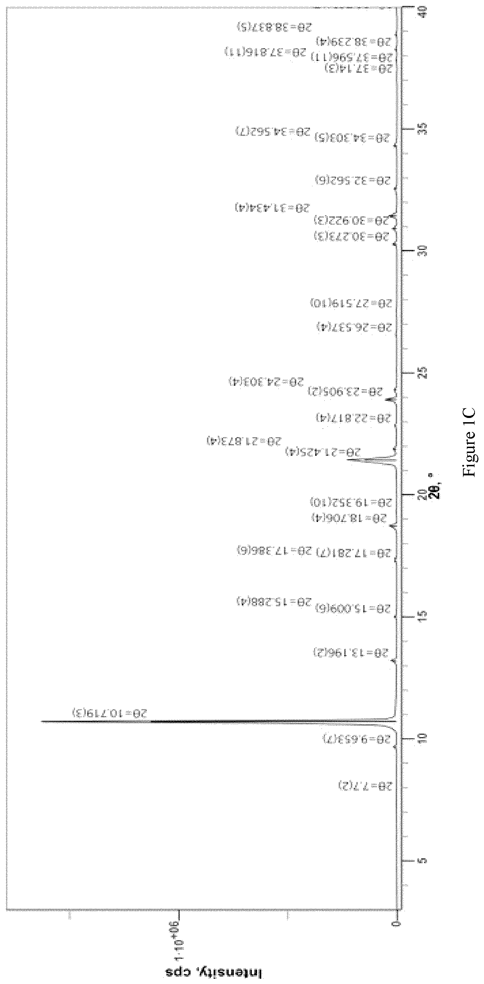

In some embodiments, the X-ray powder diffraction pattern of the complex is shown in Figure 1A, Figure 1B or Figure 1C.

In some embodiments, the infrared spectrum of the complex has the following absorption bands, expressed in reciprocal wavelengths (cm⁻¹) (±2 cm⁻¹): 1715, 1452, 1414, 1368, 1306, 1217, 1148, 1094, 1057, 980, 853, 710, 573, 467.

In some embodiments, the X-ray powder diffraction pattern of the complex comprises peaks at diffraction angles (2θ) of 6.9°±0.2°, 10.7°±0.2°, 18.1°±0.2°, 22.3°±0.2°, 23.0±0.2°, 26.8°±0.2°, and 31.2°±0.2°.

In some embodiments, the X-ray powder diffraction pattern of the complex further comprises one or more peaks at diffraction angles (2θ) of 17.2°±0.2°, 21.6°±0.2°, 25.8°±0.2°.

In some embodiments, the X-ray powder diffraction pattern of the complex comprises peaks at diffraction angles (2θ) of 7.7°±0.2°, 10.7°±0.2°, 13.2°±0.2°, 18.7±0.2°, 21.4°±0.2°, 31.4°±0.2°, and 32.6°±0.2°.

In some embodiments, the X-ray powder diffraction pattern of the complex further comprises one or more peaks at diffraction angles (2θ) of 15.0°±0.2°, 23.9°±0.2°, 34.3°±0.2°.

In some embodiments, the complex is prepared as food, beverage, supplement or pharmaceutical preparation.

In another aspect, the present invention provides a 3-hydroxybutyric acid · sodium 3-hydroxybutyrate complex, which is obtained by the following method:
(1) a substance A is obtained by one of the following methods: mixing 3-hydroxybutyric acid and sodium 3-hydroxybutyrate; or adding 3-hydroxybutyric acid into an aqueous solution of an alkaline Na compound, stirring, removing water, and evaporating to near dryness; or reacting alkyl 3-hydroxybutyrate with water under heating in the presence of a catalyst, cooling and filtering, adding an aqueous solution of an alkaline Na compound to the filtrate, distilling under reduced pressure to remove water, and evaporating to near dryness;
(2) adding one or more solvents selected from the following into the substance A obtained in step (1): water, THF, DMF, DMSO, DMAC, alcohol, halogenated hydrocarbon, ketone, ester, stirring and cooling, preferably to 0-20°C or 0-10°C, to precipitate a solid;
(3) filtering out the solid and drying, preferably at 35-65°C, to obtain the complex.

In some embodiments, in step (2), the alcohol is methanol, ethanol, isopropanol, n-butanol, preferably ethanol, isopropanol; the halogenated hydrocarbon is chlorobenzene, dichlorobenzene, dichloromethane, preferably dichloromethane; the ketone is acetone, methyl butanone, methyl isobutyl ketone, preferably acetone; the ester is ethyl acetate, isopropyl acetate, n-butyl acetate, isobutyl acetate, preferably ethyl acetate.

In some embodiments, in step (1), the alkaline Na compound can be NaOH, Na₂CO₃, NaHCO₃, NaOMe, NaOAc or NaOCHO, preferably NaOH; the alkyl 3-hydroxybutyrate can be methyl 3-hydroxybutyrate, ethyl 3-hydroxybutyrate, propyl 3-hydroxybutyrate, butyl 3-hydroxybutyrate, preferably methyl 3-hydroxybutyrate.

In some embodiments, the complex is R-3-hydroxybutyric acid · sodium R-3-hydroxybutyrate and/or S-3-hydroxybutyric acid · sodium S-3-hydroxybutyrate.

In some embodiments, the complex is in crystalline form.

In another aspect, the present invention provides a method for preparing the above-mentioned complex, including the following steps:
(1) a substance A is obtained by one of the following methods: mixing 3-hydroxybutyric acid and sodium 3-hydroxybutyrate; or adding 3-hydroxybutyric acid into an aqueous solution of an alkaline Na compound, stirring, removing water, and evaporating to near dryness; or reacting alkyl 3-hydroxybutyrate with water under heating in the presence of a catalyst, cooling and filtering, adding an aqueous solution of an alkaline Na compound to the filtrate, distilling under reduced pressure to remove water, and evaporating to near dryness;
(2) adding one or more solvents selected from the following into the substance A obtained in step (1): water, THF, DMF, DMSO, DMAC, alcohol, halogenated hydrocarbon, ketone, ester, stirring and cooling, preferably to 0-20°C or 0-10°C, to precipitate a solid;
(3) filtering out the solid and drying, preferably at 35-65°C, to obtain the complex.

In some embodiments, in step (2), the alcohol is methanol, ethanol, isopropanol, n-butanol, preferably ethanol, isopropanol; the halogenated hydrocarbon is chlorobenzene, dichlorobenzene, dichloromethane, preferably dichloromethane; the ketone is acetone, methyl butanone, methyl isobutyl ketone, preferably acetone; the ester is ethyl acetate, isopropyl acetate, n-butyl acetate, isobutyl acetate, preferably ethyl acetate.

In some embodiments, in step (1), the alkaline Na compound can be NaOH, Na₂CO₃, NaHCO₃, NaOMe, NaOAc or NaOCHO, preferably NaOH; the alkyl 3-hydroxybutyrate can be methyl 3-hydroxybutyrate, ethyl 3-hydroxybutyrate, propyl 3-hydroxybutyrate, butyl 3-hydroxybutyrate, preferably methyl 3-hydroxybutyrate.

In another aspect, the present invention provides a composition comprising an effective amount of the above-mentioned complex, and a pharmaceutically acceptable carrier.

In some embodiments, the composition is used as a ketogenic substance.

In some embodiments, the composition is prepared as food, beverage, supplement or pharmaceutical preparation.

In another aspect, the present invention provides use of the complex, the complex is used for preparing a ketogenic substance for increasing or maintaining blood ketone level of a subject.

In some embodiments, the ketogenic substance can be used as nutritional supplement, energy therapy, medical treatment or strength and/or endurance exercise supplement.

In another aspect, the present invention provides use of a composition in preparing a ketogenic substance for increasing or maintaining blood ketone level of a subject, the composition comprises the complex of the invention, and a pharmaceutically acceptable carrier.

In some embodiments, the ketogenic substance is nutritional supplement, energy therapy, medical treatment or strength and/or endurance exercise supplement.

Compared with the prior art, the complex of the invention has the beneficial effects that the complex of the invention has no bad smell and effectively avoids the problems of acidity, hygroscopicity, salt load, intestinal side effects, electrolyte imbalance and the like; and compared with other exogenous ketones, the complex has excellent adaptability. When administered to a subject, the complex shows a better comprehensive effect than acid or salt alone or simply physically mixed components. The complex of the invention solves the problems of strong acidity, intestinal side effects and high hygroscopicity of BHB acid; and at the same time solves the problem of electrolyte imbalance caused by high salt load of BHB salt, and there is no problem of uneven physical mixing, so it can be widely used as a ketogenic substance in dietary supplements or food fields.

### BRIEF DESCRIPTION OF THE DRAWINGS

Figure 1A shows an XRPD pattern of the R-3-hydroxybutyric acid · sodium R-3-hydroxybutyrate complex of the present invention.
Figure 1B shows an XRPD pattern of the 3-hydroxybutyric acid · sodium 3-hydroxybutyrate complex of the present invention.
Figure 1C shows an XRPD pattern of the S-3-hydroxybutyric acid · sodium S-3-hydroxybutyrate complex of the present invention.
Figure 2 shows an infrared spectrogram (IR) of the 3-hydroxybutyric acid · sodium 3-hydroxybutyrate complex of the present invention.
Figure 3A shows a Raman spectrogram of the R-3-hydroxybutyric acid · sodium R-3-hydroxybutyrate complex of the present invention.
Figure 3B shows a Raman spectrogram of the 3-hydroxybutyric acid · sodium 3-hydroxybutyrate complex of the present invention.
Figure 3C shows a Raman spectrogram of the S-3-hydroxybutyric acid · sodium S-3-hydroxybutyrate complex of the present invention.
Figure 4A shows a TGA diagram of the R-3-hydroxybutyric acid · sodium R-3-hydroxybutyrate complex of the present invention.
Figure 4B shows a TGA diagram of the 3-hydroxybutyric acid · sodium 3-hydroxybutyrate complex of the present invention.
Figure 4C shows a TGA diagram of the S-3-hydroxybutyric acid · sodium S-3-hydroxybutyrate complex of the present invention.
Figure 5A shows a DSC thermogram of the R-3-hydroxybutyric acid · sodium R-3-hydroxybutyrate complex of the present invention.
Figure 5B shows a DSC thermogram of the 3-hydroxybutyric acid · sodium 3-hydroxybutyrate complex of the present invention.
Figure 5C shows a DSC thermogram of the S-3-hydroxybutyric acid · sodium S-3-hydroxybutyrate complex of the present invention.

### DETAILED DESCRIPTION OF THE INVENTION

Reference will now be made in detail to the preferred embodiments of the invention, examples of which are further illustrated. While the invention will be described in conjunction with the preferred embodiments, it will be understood that they are not intended to limit the invention to these embodiments. On the contrary, the invention is intended to cover alternatives, modifications, and equivalents, which may be included within the spirit and scope of the invention as defined by the claims. Furthermore, in the detailed description of the present invention, numerous specific details are set forth in order to provide a thorough understanding of the present invention. However, it will be obvious to one of ordinary skill in the art that the present invention may be practiced without these specific details. In other instances, well known methods, procedures, components, and other features have not been described in detail as not to unnecessarily obscure aspects of the present invention.

The 3-hydroxybutyric acid · sodium 3-hydroxybutyrate complex of the invention comprises 3-hydroxybutyric acid and sodium 3-hydroxybutyrate in any suitable ratio, and the complex can be hydrate with fixed water ratio, non-hydrate, and corresponding crystal forms.

As used herein, the term "or" is meant to include both "and" and "or." In other words, the term "or" may also be replaced with "and/or."

As used herein, the singular forms "a," "an" and "the" are intended to include the plural forms as well, unless the context clearly indicates otherwise.

As used herein, the term "comprise" or "include" and their conjugations, refer to a situation wherein said terms are used in their non-limiting sense to mean that items following the word are included, but items not specifically mentioned are not excluded. It also encompasses the more limiting verb 'to consist essentially of and 'to consist of'.

As use herein, the terms "about" and "approximately" provide numerical flexibility by providing endpoints where a given value can be "slightly above" or "less than". The flexibility of this term can be determined by specific variables and based on experience and related descriptions herein within the knowledge of those skilled in the art.

β-hydroxybutyrate, also known as 3-hydroxybutyrate, βHB, or BHB, refers to a compound with the general formula CH₃CH₂OHCH₂COOH. "Beta-hydroxybutyrate derivative" represents a compound having chemical structure shown below, where X can be hydrogen, metal ion, amino cation (e.g., amino acid), and the like:

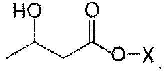

When X is a hydrogen, the compound is beta-hydroxybutyric acid. When X is a metal ion or an amino cation, the compound is a beta-hydroxybutyrate salt. The foregoing compounds can be in any desired physical form, such as crystalline, powder, solid, liquid, solution, suspension, or gel.

As used herein, the term "administration" refers to the process of delivering a disclosed composition or active ingredient to a subject. The complexes of the invention can be administered in a variety of suitable ways, including orally, intragastrically, and parenterally (e.g., intravenous and intraarterial as well as other suitable parenteral routes), and the like, so as to exert the desired effects. The complex of the invention may be administered to a subject in an effective dosages and/or in frequencies to induce or sustain ketosis. In some embodiments, a single dose will include an amount of about 1-50 grams, or about 2-40 grams, or about 5-30 grams, or about 10-20 grams, about 0.5-25 grams, or about 0.75-20 grams, or about 1-15 grams, or about 1.5-12 grams. In some embodiments, multiple doses of complex are administered over a period of time. The frequency of administration of the complex can vary depending on any of a variety of factors, such as timing of treatment from previous treatments, objectives of the treatment, and the like. The duration of administration of the complex (e.g., the period of time over which the agent is administered), can vary depending on any of a variety of factors, including subject response, desired effect of treatment, etc.

As used herein, the term "effective amount" refers to the amount required to achieve an effect as taught herein. The amount to be administered can vary according to factors such as the degree of susceptibility of the individual, the age, sex, and weight of the individual, idiosyncratic responses of the individual, and the like. In accordance with the present disclosure, a suitable single dose size is a dose that is capable of achieve the above effects when administered one or more times over a suitable time period.

As used herein, the term "pharmaceutically acceptable" means pharmaceutically, physiologically, alimentarily, and/or nutritionally acceptable, and refers to those compositions or combinations of agents, materials, or compositions, and/or their dosage forms, which are within the scope of sound medical judgment, suitable for use in contact with the tissues of human beings and animals without excessive toxicity, irritation, allergic response, or other problem or complication, commensurate with a reasonable benefit/risk ratio.

In some embodiments, because beta-hydroxybutyrate may be R-beta-hydroxybutyrate enantiomer, which is endogenously produced by a mammal during ketosis, administering the R-beta-hydroxybutyrate enantiomer to a subject provides an additional quantity and/or increased blood plasma level that can be immediately utilized by the body, such as for producing energy (e.g., as an alternative energy source to glucose).

The complexes and/or compositions of the present invention may be used for preparing a ketogenic substance for increasing or maintaining blood ketone level of a subject, and increasing ketone body level in a subject, including inducing and/or sustaining a state of elevated ketone bodies at a desired level, such as ketosis, in a subject to which it is administered. "Ketosis" refers to a subject having blood ketone levels within the range of about 0.5 mmol/L and about 16 mmol/L in a subject. Ketosis may improve mitochondrial function, decrease reactive oxygen species production, reduce inflammation and increase the activity of neurotrophic factors. "Keto-adaptation" refers to prolonged nutritional ketosis (>1 week) to achieve a sustained nonpathological "mild ketosis" or "therapeutic ketosis." In some cases, "elevated ketone body level" may not mean that a subject is in a state of "clinical ketosis" but nevertheless has an elevated supply of ketones for producing energy and/or for carrying out other beneficial effects of ketone bodies.

The administration of the complexes and/or compositions of the present invention can increase or maintain blood ketone level of a subject, and as a ketogenic substance, produce one or more desired effects, including but not limited to appetite suppression, weight loss, fat loss, reduced blood glucose level, improved mental alertness, increased physical energy, improved cognitive function, reduction in traumatic brain injury, reduction in effect of diabetes, improvement of neurological disorder, reduction of cancer, reduction of inflammation, anti-aging, antiglycation, reduction in epileptic seizer, improved mood, increased strength, increased muscle mass, or improved body composition.

In some embodiments, the complex of the present invention can be prepared into a composition together with an alimentarily or pharmaceutically acceptable carrier. In the present invention, the application form of providing composition involves liquid or solid fillers, diluents, excipients, solvents or encapsulate materials. Each carrier must be "acceptable", which means that it is compatible with other components of the composition and harmless to the subject, that is, suitable for consumption or nutritionally acceptable. The carriers include non-toxic and compatible substances commonly used in health foods, dietary supplements and pharmaceutical preparations, such as sugar, starch, cellulose and its derivatives, powdered tragacanth, malt, gelatin, talc, oil, diol, polyol, ester, agar, alginic acid, pyrogen free water, isotonic saline, etc.

In some embodiments, the complexes of the present invention can be administered together with other supplements, such as vitamins, minerals, nootropics, and others known in the art. Examples of vitamins, minerals and herbal supplements that can be added to the ketogenic compositions include one or more of vitamin A, vitamin C, vitamin D3, vitamin E, niacin, vitamin B6, folic acid, 5-MTHF, vitamin B12, iodine, zinc, copper, manganese, chromium, caffeine, theobromine, theacrine, methylliberine, huperzine A, epicatechins, and enzymes

In some embodiments, the complexes of the present invention may be provided as a solid or powder form. The compositions in such solid form may be formulated so as to provide for sufficient ease of handling and manufacturability. The complexes may be provided as a liquid, such as in the form of a shot or mouth spray for fast delivery and absorption. Liquid forms may include one or more liquid carriers, such as water, ethanol, glycerin, propylene glycol, 1, 3-propandiol, and the like.

In some embodiments, the complex of the present invention can be used as suppository, tablet, pill, granule, powder, film, capsule, beverage, aerosol, alcohol, tincture, tonic, liquid suspension or syrup.

The complex and/or composition of the present invention can be prepared as food and beverage products for human consumption, as well as nutritional supplements, energy therapy, medical treatment or strength and/or endurance exercise supplements as ketogenic substances, thereby providing a dietary source of exogenous ketones and raising or maintaining blood ketone level of a subject. The obtained product can show balance effects of reduced acidity, lower hygroscopicity, better taste, better palatability, uniform appearance and good ketogenic effect, and has no problems of intestinal side effects, electrolyte imbalance and high salt load.

The following examples are illustrative of select embodiments of the present invention and are not meant to limit the scope of the invention.

### Preparation of complexes of the present invention

### Example 1. Preparation of R-3-hydroxybutyric acid · sodium R-3-hydroxybutyrate complex

110 g of R-3-hydroxybutyric acid, 110 g of sodium R-3-hydroxybutyrate, 440 mL dichloromethane were added into a 1-L reaction bottle, heated to 40°C. It was stirred and dissolved, cooled to 0-10°C; the solid was precipitated, filtered, and dried at 40°C, to obtain 150 g of R-3-hydroxybutyric acid · sodium R-3-hydroxybutyrate complex.

### Example 2. Preparation of R-3-hydroxybutyric acid · sodium R-3-hydroxybutyrate complex

104 g of R-3-hydroxybutyric acid, 116 g of sodium R-3-hydroxybutyrate, 440 mL acetone were added into a 1-L reaction bottle, heated to 60°C. It was stirred and dissolved, cooled to 0-10°C; the solid was precipitated, filtered, and dried at 55°C, to obtain 140 g of R-3-hydroxybutyric acid · sodium R-3-hydroxybutyrate complex.

### Example 3. Preparation of R-3-hydroxybutyric acid · sodium R-3-hydroxybutyrate complex

200 mL water and 20 g sodium hydroxide were added into a 1-L reaction bottle. It was stirred and dissolved, cooled to below 25°C, and then 104 g of R-3-hydroxybutyric acid was added. It was stirred for half an hour, and distilled under reduced pressure to remove water. After evaporation to near dryness, 200 mL of dichloromethane was added. It was stirred, cooled to 0-10°C; the solid was precipitated, filtered, and dried at 40°C, to obtain 75 g of R-3-hydroxybutyric acid · sodium R-3-hydroxybutyrate complex.

### Example 4. Preparation of R-3-hydroxybutyric acid · sodium R-3-hydroxybutyrate complex

200 mL water and 20 g sodium hydroxide were added into a 1-L reaction bottle. It was stirred and dissolved, cooled to below 25°C, and then 104 g of R-3-hydroxybutyric acid was added. It was stirred for half an hour, and distilled under reduced pressure to remove water. After evaporation to near dryness, 200 mL of acetone was added. It was stirred, cooled to 0-10°C; the solid was precipitated, filtered, and dried at 55°C, to obtain 98 g of R-3-hydroxybutyric acid · sodium R-3-hydroxybutyrate complex.

### Example 5. Preparation of R-3-hydroxybutyric acid · sodium R-3-hydroxybutyrate complex

60 g of methyl R-3-hydroxybutyrate, 360 mL water and 24 g of catalyst were added into a 1-L reaction bottle, heated at 90-95°C for 24 hours until the reaction was complete. It was cooled to room temperature, and the catalyst was filtered off. 50 mL aqueous solution of 9 g of sodium hydroxide was added to the filtrate, and the water was distilled off under reduced pressure. After evaporation to near dryness, 100 mL of dichloromethane was added. It was stirred, cooled to 0-10°C; the solid was precipitated, filtered, and dried at 40°C, to obtain 40 g of R-3-hydroxybutyric acid · sodium R-3-hydroxybutyrate complex.

### Example 6. Preparation of R-3-hydroxybutyric acid · sodium R-3-hydroxybutyrate complex

60 g of methyl R-3-hydroxybutyrate, 360 mL water and 24 g of catalyst were added into a 1-L reaction bottle, heated at 90-95°C for 24 hours until the reaction was complete. It was cooled to room temperature, and the catalyst was filtered off. 50 mL aqueous solution of 9 g of sodium hydroxide was added to the filtrate, and the water was distilled off under reduced pressure. After evaporation to near dryness, 100 mL of acetone was added. It was stirred, cooled to 0-10°C; the solid was precipitated, filtered, and dried at 55°C, to obtain 50 g of R-3-hydroxybutyric acid · sodium R-3-hydroxybutyrate complex.

### Example 7. Preparation of 3-hydroxybutyric acid · sodium 3-hydroxybutyrate complex

110 g of 3-hydroxybutyric acid, 110 g of sodium R-3-hydroxybutyrate, and 440 mL dichloromethane were added into a 1-L reaction bottle, heated to 40°C. It was stirred and dissolved, cooled to 0-10°C; the solid was precipitated, filtered, and dried at 40°C, to obtain 155 g of 3-hydroxybutyric acid · sodium 3-hydroxybutyrate complex.

### Example 8. Preparation of 3-hydroxybutyric acid · sodium 3-hydroxybutyrate complex

200 mL water and 20 g of sodium hydroxide were added into a 1-L reaction bottle. It was stirred and dissolved, cooled to below 25°C, and then 104 g of 3-hydroxybutyric acid was added. It was stirred for half an hour, and distilled under reduced pressure to remove water. After evaporation to near dryness, 200 mL of acetone was added. It was stirred, cooled to 0-10°C; the solid was precipitated, filtered, and dried at 60°C, to obtain 102 g of 3-hydroxybutyric acid · sodium 3-hydroxybutyrate complex.

### Example 9. Preparation of 3-hydroxybutyric acid · sodium 3-hydroxybutyrate complex

60 g of methyl 3-hydroxybutyrate, 360 mL water and 24 g of catalyst were added into a 1-L reaction bottle, heated at 90-100°C for 24 hours until the reaction was complete. It was cooled to room temperature, and the catalyst was filtered off. 50 mL aqueous solution of 9 g of sodium hydroxide was added to the filtrate, and the water was distilled off under reduced pressure. After evaporation to near dryness, 100 mL of acetone was added. It was stirred, cooled to 0-10°C; the solid was precipitated, filtered, and dried at 60°C, to obtain 42 g of 3-hydroxybutyric acid · sodium 3-hydroxybutyrate complex.

### Example 10. Preparation of S-3-hydroxybutyric acid · sodium S-3-hydroxybutyrate complex

100 g of S-3-hydroxybutyric acid, 100 g of sodium S-3-hydroxybutyrate, 400 mL dichloromethane were added into a 1-L reaction bottle, heated to 40°C. It was stirred and dissolved, cooled to 0-10°C; the solid was precipitated, filtered, and dried at 40°C, to obtain 130 g of S-3-hydroxybutyric acid · sodium S-3-hydroxybutyrate complex.

### Example 11. Preparation of S-3-hydroxybutyric acid · sodium S-3-hydroxybutyrate complex

104 g of S-3-hydroxybutyric acid, 116 g of sodium S-3-hydroxybutyrate, 440 mL acetone were added into a 1-L reaction bottle, heated to 60°C. It was stirred and dissolved, cooled to 0-10°C; the solid was precipitated, filtered, and dried at 55°C, to obtain 138 g of S-3-hydroxybutyric acid · sodium S-3-hydroxybutyrate complex.

### Example 12. Preparation of S-3-hydroxybutyric acid · sodium S-3-hydroxybutyrate complex

200 mL water and 20 g of sodium hydroxide were added into a 1-L reaction bottle. It was stirred and dissolved, cooled to below 25°C, and then 104 g of S-3-hydroxybutyric acid was added. It was stirred for half an hour, and distilled under reduced pressure to remove water. After evaporation to near dryness, 200 mL of dichloromethane was added. It was stirred, cooled to 0-10°C; the solid was precipitated, filtered, and dried at 40°C, to obtain 73 g of S-3-hydroxybutyric acid · sodium S-3-hydroxybutyrate complex.

### Example 13. Preparation of S-3-hydroxybutyric acid · sodium S-3-hydroxybutyrate complex

200 mL water and 20 g sodium hydroxide were added into a 1-L reaction bottle. It was stirred and dissolved, cooled to below 25°C, and then 104 g of S-3-hydroxybutyric acid was added. It was stirred for half an hour, and distilled under reduced pressure to remove water. After evaporation to near dryness, 200 mL of acetone was added. It was stirred, cooled to 0-10°C; the solid was precipitated, filtered, and dried at 55°C, to obtain 98 g of S-3-hydroxybutyric acid · sodium S-3-hydroxybutyrate complex.

### Example 14. Preparation of S-3-hydroxybutyric acid · sodium S-3-hydroxybutyrate complex

60 g of methyl S-3-hydroxybutyrate, 360 mL water and 24 g of catalyst were added into a 1-L reaction bottle, heated at 90-95°C for 24 hours until the reaction was complete. It was cooled to room temperature, and the catalyst was filtered off. 50 mL aqueous solution of 9 g of sodium hydroxide was added to the filtrate, and the water was distilled off under reduced pressure. After evaporation to near dryness, 100 mL of dichloromethane was added. It was stirred, cooled to 0-10°C; the solid was precipitated, filtered, and dried at 40°C, to obtain 42 g of S-3-hydroxybutyric acid · sodium S-3-hydroxybutyrate complex.

### Example 15. Preparation of S-3-hydroxybutyric acid · sodium S-3-hydroxybutyrate complex

60 g of methyl S-3-hydroxybutyrate, 360 mL water and 24 g of catalyst were added into a 1-L reaction bottle, heated at 90-95°C for 24 hours until the reaction was complete. It was cooled to room temperature, and the catalyst was filtered off. 50 mL aqueous solution of 9 g of sodium hydroxide was added to the filtrate, and the water was distilled off under reduced pressure. After evaporation to near dryness, 100 mL of acetone was added. It was stirred, cooled to 0-10°C; the solid was precipitated, filtered, and dried at 55°C, to obtain 48 g of S-3-hydroxybutyric acid · sodium S-3-hydroxybutyrate complex.

### Characterization of complexes of the present invention

X-ray powder diffraction (XRPD), elemental analysis, Raman spectroscopy, infrared spectroscopy (IR), TGA, DSC, DVS, etc. were performed on the complexes prepared in Examples 1-15.

### Example 16. X-ray powder diffraction

X-ray powder diffraction patterns were obtained by using SmartLab 3KW X-ray powder diffractometer under the following conditions: diffraction line: Cu_K-beta (40KV, 40mA), scanning rate: 20.00 deg/min, and scanning range: 3°~ 60°. The XRPD pattern of the complex obtained in Example 1 is shown in Figure 1A, and the XRPD data is shown in Table 1A. The XRPD results of the complexes prepared in Examples 2-6 are substantially the same as those in Example 1.

**Table 1A**

| position (2θ, °) | spacing (d, Å) | relative intensity (%) |
|---|---|---|
| 9.8 | 9 | 0.62 |
| 10.9 | 8.1 | 100 |
| 13.3 | 6.6 | 1.66 |
| 15.1 | 5.9 | 0.8 |
| 17.5 | 5.1 | 2.25 |
| 18.9 | 4.7 | 0.74 |
| 21.4 | 4.1 | 5.06 |
| 21.6 | 4.1 | 16.44 |
| 22.0 | 4 | 1.05 |
| 23.0 | 3.9 | 0.95 |
| 24.0 | 3.7 | 5.25 |
| 24.4 | 3.6 | 1.57 |
| 26.8 | 3.3 | 0.99 |
| 27.8 | 3.2 | 0.78 |
| 30.5 | 2.9 | 1.6 |
| 31.1 | 2.9 | 2.31 |
| 31.5 | 2.8 | 3.95 |
| 31.9 | 2.8 | 0.25 |
| 32.7 | 2.7 | 3.02 |
| 34.5 | 2.6 | 1.64 |

The XRPD pattern of the complex obtained in Example 7 is shown in Figure 1B, and the XRPD data is shown in Table 1B. The XRPD results of the complexes prepared in Examples 8-9 are substantially the same as those in Example 7.

**Table 1B**

| position (2θ, °) | spacing (d, Å) | relative intensity (%) |
|---|---|---|
| 6.9 | 12.8 | 100 |
| 10.7 | 8.2 | 35.41 |
| 13.3 | 6.6 | 9.23 |
| 14.7 | 6.0 | 0.78 |
| 17.2 | 5.1 | 8.98 |
| 18.1 | 4.9 | 11.92 |
| 19.0 | 4.7 | 2.28 |
| 19.5 | 4.5 | 1.63 |
| 20.8 | 4.3 | 1.16 |
| 21.6 | 4.1 | 77.3 |
| 22.3 | 4.0 | 12.09 |
| 23.0 | 3.9 | 7.85 |
| 24.0 | 3.7 | 2.65 |
| 25.2 | 3.5 | 3.13 |
| 25.8 | 3.4 | 4.25 |
| 26.8 | 3.3 | 5.2 |
| 27.8 | 3.2 | 1.5 |
| 28.7 | 3.1 | 1.85 |
| 30.2 | 3.0 | 2.45 |
| 31.2 | 2.9 | 2.91 |

The XRPD pattern of the complex obtained in Example 10 is shown in Figure 1C, and the XRPD data is shown in Table 1C. The XRPD results of the complexes prepared in Examples 11-15 are substantially the same as those in Example 10.

**Table 1C**

| position (2θ, °) | spacing (d, Å) | relative intensity (%) |
|---|---|---|
| 7.7 | 11.4 | 1.91 |
| 9.7 | 9.2 | 0.69 |
| 10.7 | 8.2 | 100 |
| 13.2 | 6.7 | 1.67 |
| 15.0 | 5.9 | 0.76 |
| 17.3 | 5.1 | 0.59 |
| 18.7 | 4.7 | 3.02 |
| 21.4 | 4.1 | 22.46 |
| 21.9 | 4.1 | 1.12 |
| 22.8 | 3.9 | 0.63 |
| 23.9 | 3.7 | 3.76 |
| 26.5 | 3.4 | 0.52 |
| 27.5 | 3.2 | 0.45 |
| 30.3 | 2.9 | 1.31 |
| 31.4 | 2.8 | 2.85 |
| 32.6 | 2.7 | 1.26 |
| 34.3 | 2.6 | 1.23 |
| 37.1 | 2.4 | 0.38 |
| 38.2 | 2.4 | 0.62 |
| 38.8 | 2.3 | 0.6 |

### Example 17. Infrared spectroscopy

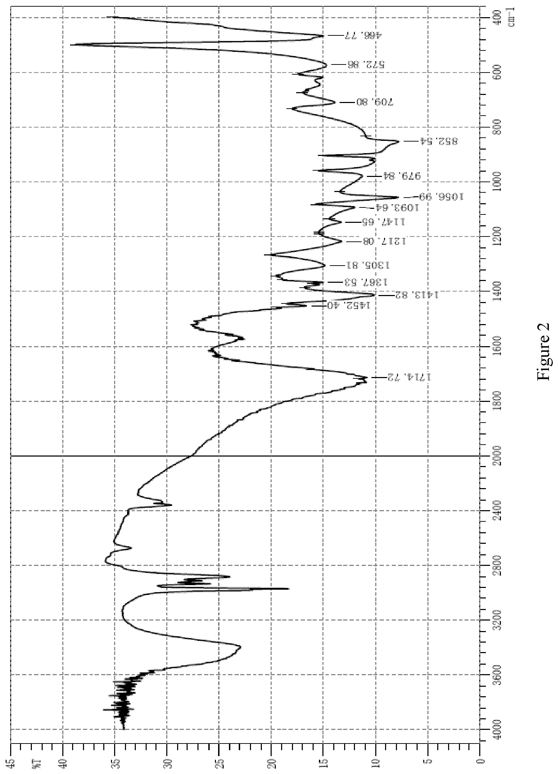

The infrared spectrum of the complex of Example 1 was analyzed by SHIMADZU's Fourier transform attenuated total reflection infrared spectrometer. Figure 2 shows an infrared spectrogram (IR) of the complex of Example 1. As shown in Figure 2, the complex has characteristic absorption peaks at 1715 cm⁻¹, 1452 cm⁻¹, 1414 cm⁻¹, 1368 cm⁻¹, 1306 cm⁻¹, 1217 cm⁻¹, 1148 cm⁻¹, 1094 cm⁻¹, 1057 cm⁻¹, 980 cm⁻¹, 853 cm⁻¹, 710 cm⁻¹, 573 cm⁻¹, 467 cm⁻¹. The IR results of the complexes prepared in Examples 2-15 are substantially the same as those in Example 1.

### Example 18. Determination of sodium content and BHB content

Using ICP-Ms, the content of sodium in the complex of Example 1 was determined according to the second method of GB 5009.268-2016, the third method of GB 5009.91-2017, and the content of 3-hydroxybutyric acid in the complex was determined by HPLC. The detection results were consistent with the structure of the complex. The results of sodium content and 3-hydroxybutyric acid content of the complex prepared in Examples 2-15 are consistent with those in Example 1.

### Example 19. NMR (¹H/¹³C) determination

¹H NMR and ¹³C NMR spectra of the complex of Example 1 were recorded in an AVIII-HD-400 spectrometer: ¹H NMR (400 MHz, DMSO-d6) δ 7.61 (s, 1H), 3.92 (h, J = 6.2 Hz, 1H), 2.17 (d, J = 6.5 Hz, 2H), 1.04 (d, J = 6.3 Hz, 3H). ¹³C NMR (101 MHz, DMSO-d6) δ 176.43, 64.21, 45.62, 23.64. The NMR (¹H/¹³C) results of the complexes prepared in Examples 2-15 are substantially the same as those in Example 1.

### Example 20. Elemental analysis

Elemental analysis was performed on the complex of Example 10: C 41.7%, H 6.5%. The elemental analysis results of other examples are also consistent with those of Example 10, and the results are consistent with the structure of 3-hydroxybutyric acid · sodium 3-hydroxybutyrate complex.

### Example 21. Raman spectroscopy

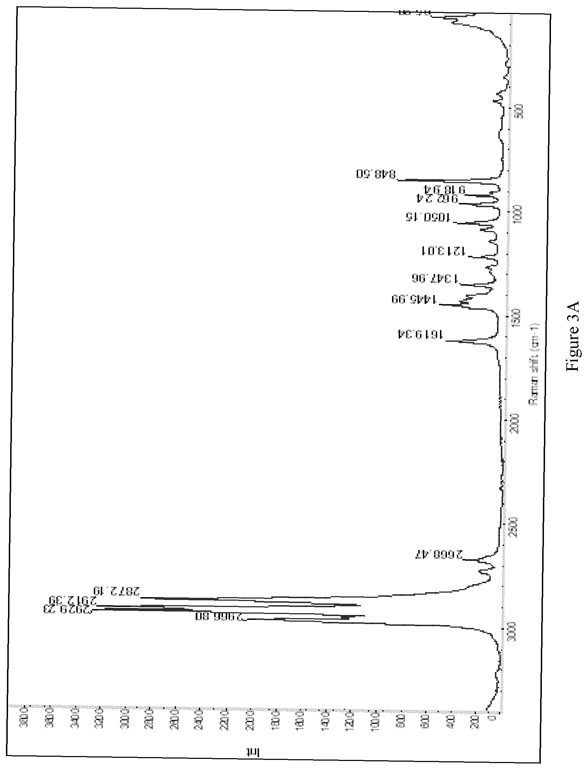

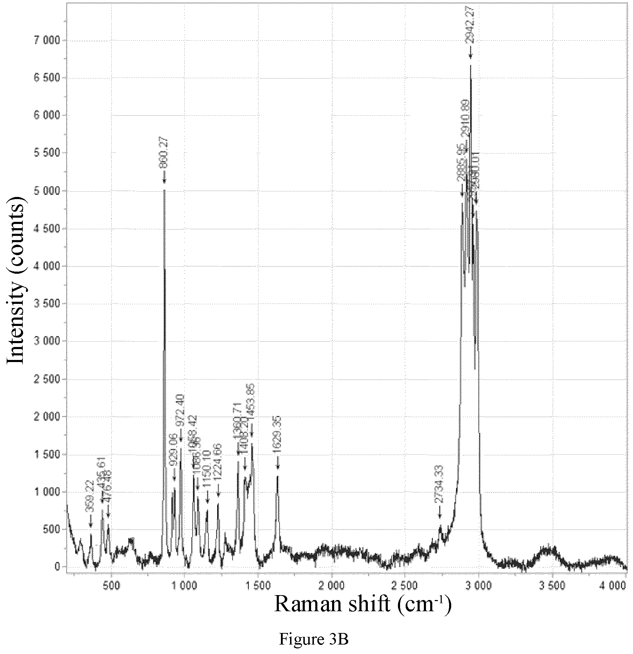

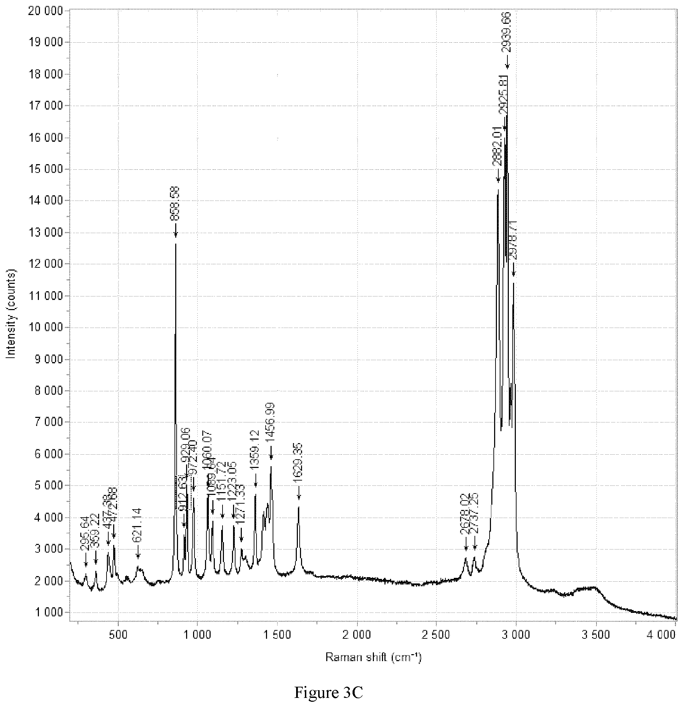

The characteristic Raman spectrum of the complex can be obtained by Raman spectroscopy. Figure 3A shows a Raman spectrogram of the complex of Example 1. The complex of Example 1 comprises the following characteristic absorption peaks at 2966.80 cm⁻¹, 2929.23 cm⁻¹, 2912.39 cm⁻¹, 2872.19 cm⁻¹, 2668.47 cm⁻¹, 1619.34 cm⁻¹, 1445.99 cm⁻¹, 1347.96 cm⁻¹, 1213.01 cm⁻¹, 1050.15 cm⁻¹, 962.24 cm⁻¹, 918.94 cm⁻¹, 845.50 cm⁻¹, 65.80 cm⁻¹, and the error margin is ±2 cm⁻¹. The Raman spectra of the complexes prepared in Examples 2-6 are substantially the same as those in Example 1. Figure 3B shows a Raman spectrogram of the complex of Example 7. The complex of Example 7 comprises the following characteristic absorption peaks at 435.61 cm⁻¹, 860.27 cm⁻¹, 929.06 cm⁻¹, 972.40 cm⁻¹, 1058.42 cm⁻¹, 1086.36 cm⁻¹, 1224.66 cm⁻¹, 1360.71 cm⁻¹, 1408.20 cm⁻¹, 1453.85 cm⁻¹, 1629.35 cm⁻¹, 2885.95 cm⁻¹, 2910.89 cm⁻¹, 2942.27 cm⁻¹, 2980.01 cm⁻¹, and the error margin is ±2 cm⁻¹. The Raman spectra of the complexes prepared in Examples 8-9 are substantially the same as those in Example 7. Figure 3C shows a Raman spectrogram of the complex of Example 10. The complex of Example 10 comprises the following characteristic absorption peaks at 472.68 cm⁻¹, 858.58 cm⁻¹, 912.63 cm⁻¹, 929.06 cm⁻¹, 972.40 cm⁻¹, 1060.07 cm⁻¹, 1089.64 cm⁻¹, 1151.72 cm⁻¹, 1223.05 cm⁻¹, 1359.12 cm⁻¹, 1456.99 cm⁻¹, 1629.35 cm⁻¹, 2882.01 cm⁻¹, 2925.81 cm⁻¹, 2939.66 cm⁻¹, 2978.71 cm⁻¹, and the error margin is ±2 cm⁻¹. The Raman spectra of the complexes prepared in Examples 11-15 are substantially the same as those in Example 10.

### Example 22. Thermogravimetric analysis (TGA)

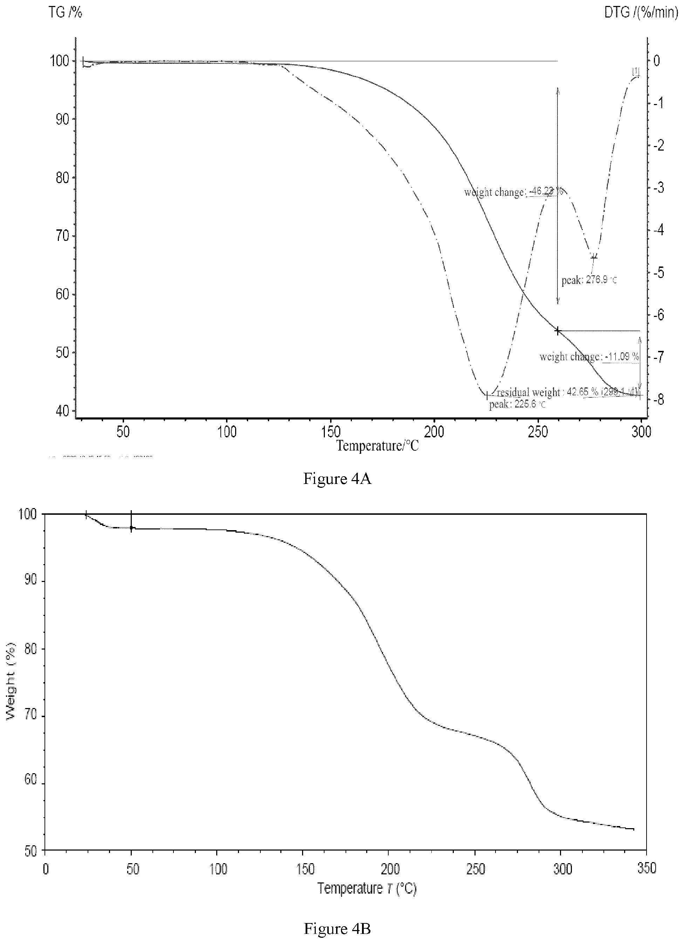

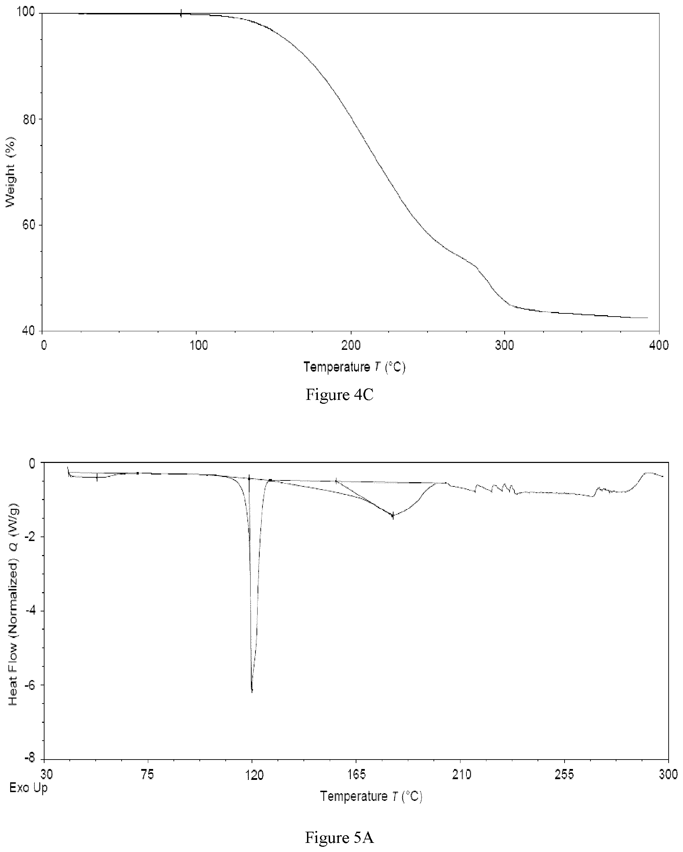

Figure 4A shows a TGA diagram of the complex of Example 1. The complex has a weight loss of 46.23% when heated from 25°C to 260°C, and 11.09% when heated from 260°C to 300°C, corresponding to the endothermic peaks at 225.6°C and 276.9°C respectively. The TGA results of the complexes prepared in Examples 2-6 are substantially the same as those in Example 1. Figure 4B shows a TGA diagram of the complex of Example 7. The complex has a weight loss of 2.02% when heated from 23.9°C to 50.0°C. The TGA results of the complexes prepared in Examples 8-9 are substantially the same as those in Example 7. Figure 4C shows a TGA diagram of the complex of Example 10. The complex has a weight loss of 0.19% when heated from 23.8°C to 90.0°C. The TGA results of the complexes prepared in Examples 11-15 are substantially the same as those in Example 10.

### Example 23. Differential scanning calorimetry (DSC)

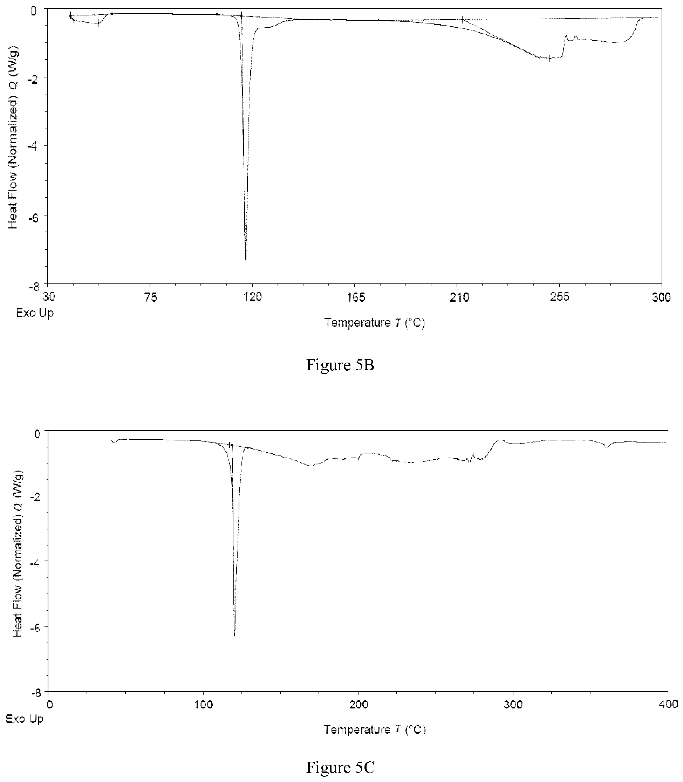

Figure 5A shows a DSC thermogram of the complex of Example 1, which contains an endothermic peak of 120.01°C±3°C. The DSC results of the complexes prepared in Examples 2-6 are substantially the same as those in Example 1. Figure 5B shows a DSC thermogram of the complex of Example 7, which contains an endothermic peak of 117.20°C±3°C. The DSC results of the complexes prepared in Examples 8-9 are substantially the same as those in Example 7. Figure 5C shows a DSC thermogram of the complex of Example 10, which contains an endothermic peak of 120.23°C±3°C. The DSC results of the complexes prepared in Examples 11-15 are substantially the same as those in Example 10.

### Determination of properties of complexes of the present invention

### Example 24. Determination of moisture contents of complexes of the present invention

For R-3-hydroxybutyric acid, sodium R-3-hydroxybutyrate, Mixture of R-3-hydroxybutyric acid and sodium R-3-hydroxybutyrate, as well as R-3-hydroxybutyric acid · sodium R-3-hydroxybutyrate complex of Examples 1-6, under a certain condition, KF moisture meter was used to determine the moisture contents at different time points. The experimental results are shown in Table 2A.

**Table 2A**

| Condition | Sample | moisture content (%) | | | | | | | |
|---|---|---|---|---|---|---|---|---|---|
| | | 30 min | 1h | 2h | 4h | 6h | 7h | 11h | 22 h |
| temperature 23°C | R-3-hydroxybutyric acid | 1.13 | 2.25 | 3.96 | 6.46 | 9.52 | 10.56 | 17.22 | 33.34 |
| | Sodium R-3-hydroxybutyrate | 1.04 | 2.01 | 3.39 | 5.23 | 7.35 | 8.04 | 12.68 | 24.68 |
| | Mixture of R-3-hydroxybutyric acid and sodium R-3-hydroxybutyrate | 1.29 | 2.39 | 3.79 | 5.43 | 6.96 | 7.44 | 10.88 | 19.85 |
| humidity 92.5% | | | | | | | | | |
| | R-3-hydroxybutyric acid · sodium R-3-hydroxybutyrate complex | 0.54 | 1.02 | 1.96 | 3.26 | 4.89 | 5.39 | 8.65 | 17.45 |

For 3-hydroxybutyric acid, sodium 3-hydroxybutyrate, mixture of 3-hydroxybutyric acid and sodium 3-hydroxybutyrate, as well as 3-hydroxybutyric acid · sodium 3-hydroxybutyrate complex of Examples 7-9, under a certain condition, KF moisture meter was used to determine the moisture contents at different time points. The experimental results are shown in Table 2B.

**Table 2B**

| Condition | Sample | moisture content (%) | | | | | | | |
|---|---|---|---|---|---|---|---|---|---|
| | | 30 min | 1h | 2h | 4h | 6h | 7h | 11h | 22 h |
| temperature 23°C | 3-hydroxybutyric acid | 1.32 | 2.37 | 4.15 | 6.59 | 9.68 | 10.71 | 17.33 | 33.51 |
| | Sodium 3-hydroxybutyrate | 1.15 | 2.19 | 3.62 | 5.35 | 7.54 | 8.35 | 12.86 | 24.95 |
| | Mixture of 3-hydroxybutyric acid and sodium 3-hydroxybutyrate | 1.43 | 2.46 | 3.96 | 5.61 | 7.19 | 7.82 | 11.38 | 20.33 |
| humidity 92.5% | | | | | | | | | |
| | 3-hydroxybutyric acid · sodium 3-hydroxybutyrate complex | 0.61 | 1.18 | 2.05 | 3.38 | 4.99 | 5.52 | 8.73 | 17.63 |

For S-3-hydroxybutyric acid, sodium S-3-hydroxybutyrate, mixture of S-3-hydroxybutyric acid and sodium S-3-hydroxybutyrate, as well as S-3-hydroxybutyric acid · sodium S-3-hydroxybutyrate complex of Examples 10-12, under a certain condition, KF moisture meter was used to determine the moisture contents at different time points. The experimental results are shown in Table 2C.

**Table 2C**

| Condition | Sample | moisture content (%) | | | | | | | |
|---|---|---|---|---|---|---|---|---|---|
| | | 30 min | 1h | 2h | 4h | 6h | 7h | 11h | 22 h |
| temperature 23°C | S-3-hydroxybutyric acid | 1.23 | 2.28 | 4.05 | 6.32 | 9.53 | 10.62 | 17.08 | 32.95 |
| | Sodium S-3-hydroxybutyrate | 1.09 | 2.07 | 3.49 | 5.32 | 7.46 | 8.15 | 12.73 | 24.53 |
| | Mixture of S-3-hydroxybutyric acid and sodium S-3-hydroxybutyrate | 1.42 | 2.43 | 3.98 | 5.87 | 7.23 | 7.64 | 11.88 | 18.95 |
| humidity 92.5% | | | | | | | | | |
| | S-3-hydroxybutyric acid · sodium S-3-hydroxybutyrate complex | 0.71 | 1.38 | 1.85 | 3.28 | 4.79 | 5.32 | 8.43 | 16.93 |

The above results show that the moisture content of R-3-hydroxybutyric acid · sodium R-3-hydroxybutyrate complex is significantly lower than that of R-3-hydroxybutyric acid, sodium R-3-hydroxybutyrate, and mixture of R-3-hydroxybutyric acid and sodium R-3-hydroxybutyrate; the moisture content of 3-hydroxybutyric acid · sodium 3-hydroxybutyrate complex is significantly lower than that of 3-hydroxybutyric acid, sodium 3-hydroxybutyrate, and mixture of 3-hydroxybutyric acid and sodium 3-hydroxybutyrate; the moisture content of S-3-hydroxybutyric acid · sodium S-3-hydroxybutyrate complex is significantly lower than that of S-3-hydroxybutyric acid, sodium S-3-hydroxybutyrate, and mixture of S-3-hydroxybutyric acid and sodium S-3-hydroxybutyrate, which can increase the application scenarios of complex products. Because solids of R-3-hydroxybutyric acid, 3-hydroxybutyric acid, and S-3-hydroxybutyric acid have strong hygroscopicity and are easy to deliquesce, they cannot be well used in the field of solid preparations, which greatly limits their application in the fields of solid nutrition and dietary supplements. Whereas, in the present invention, the hygroscopicity of R-3-hydroxybutyric acid · sodium R-3-hydroxybutyrate complex is obviously superior to that of R-3-hydroxybutyric acid, sodium R-3-hydroxybutyrate, and mixture of R-3-hydroxybutyric acid and sodium R-3-hydroxybutyrate; the hygroscopicity of 3-hydroxybutyric acid · sodium 3-hydroxybutyrate complex is obviously superior to that of 3-hydroxybutyric acid, sodium 3-hydroxybutyrate, and mixture of 3-hydroxybutyric acid and sodium 3-hydroxybutyrate; the hygroscopicity of S-3-hydroxybutyric acid · sodium S-3-hydroxybutyrate complex is obviously superior to that of S-3-hydroxybutyric acid, sodium S-3-hydroxybutyrate, and mixture of S-3-hydroxybutyric acid and sodium S-3-hydroxybutyrate. In addition, simple mixture of acid and sodium salt is prone to uneven mixing. Therefore, the application range of complex is wider, especially suitable for the preparation and application of solid preparations.

### Example 25. Stability of complexes of the present invention

For R-3-hydroxybutyric acid, sodium R-3-hydroxybutyrate, mixture of R-3-hydroxybutyric acid and sodium R-3-hydroxybutyrate, as well as R-3-hydroxybutyric acid · sodium R-3-hydroxybutyrate complex of Examples 1-6, the stability was measured at 60°C, 70°C and 80°C, and the experimental results are shown in Table 3A.

**Table 3A**

| Sample | Stability | | | | | |
|---|---|---|---|---|---|---|
| | 30°C | 40°C | 50°C | 60°C | 70°C | 80°C |
| R-3-hydroxybutyri c acid | unchanged | partially melted | melted | melted | melted | melted |
| Sodium R-3-hydroxybutyr ate | unchanged | unchanged | unchanged | unchanged | unchanged | unchanged |
| Mixture of R-3-hydroxybutyri c acid and sodium R-3-hydroxybutyr ate | unchanged | partially melted | partially melted | partially melted | partially melted | melted |
| R-3-hydroxybutyri c acid · sodium R-3-hydroxybutyr ate complex | unchanged | unchanged | unchanged | unchanged | unchanged | unchanged |

For 3-hydroxybutyric acid, sodium 3-hydroxybutyrate, mixture of 3-hydroxybutyric acid and sodium 3-hydroxybutyrate, as well as 3-hydroxybutyric acid · sodium 3-hydroxybutyrate complex of Examples 7-9, the stability was measured at 60°C, 70°C and 80°C, and the experimental results are shown in Table 3B.

**Table 3B**

| Sample | Stability | | | | | |
|---|---|---|---|---|---|---|
| | 30°C | 40°C | 50°C | 60°C | 70°C | 80°C |
| 3-hydroxybutyric acid | unchanged | partially melted | melted | melted | melted | melted |
| sodium 3 -hydroxybutyrate | unchanged | unchanged | unchanged | unchanged | unchanged | unchanged |
| Mixture of 3-hydroxybutyric acid and sodium 3-hydroxybutyrate | unchanged | partially melted | partially melted | partially melted | partially melted | melted |
| 3-hydroxybutyric acid · sodium 3-hydroxybutyrate complex | unchanged | unchanged | unchanged | unchanged | unchanged | unchanged |

For S-3-hydroxybutyric acid, sodium S-3-hydroxybutyrate, mixture of S-3-hydroxybutyric acid and sodium S-3-hydroxybutyrate, as well as S-3-hydroxybutyric acid · sodium S-3-hydroxybutyrate complex of Examples 10-12, the stability was measured at 60°C, 70°C and 80°C, and the experimental results are shown in Table 3C.

**Table 3C**

| Sample | Stability | | | | | |
|---|---|---|---|---|---|---|
| | 30°C | 40°C | 50°C | 60°C | 70°C | 80°C |
| S-3-hydroxybutyri c acid | unchanged | partially melted | melted | melted | melted | melted |
| Sodium S-3-hydroxybutyra te | unchanged | unchanged | unchanged | unchanged | unchanged | unchanged |
| Mixture of S-3-hydroxybutyri c acid and sodium S-3-hydroxybutyra te | unchanged | partially melted | partially melted | partially melted | partially melted | melted |
| S-3-hydroxybutyri c acid · sodium S-3-hydroxybutyra te complex | unchanged | unchanged | unchanged | unchanged | unchanged | unchanged |

The above results show that the stability of R-3-hydroxybutyric acid · sodium R-3-hydroxybutyrate complex is very high, which is significantly higher than that of R-3-hydroxybutyric acid, and mixture of R-3-hydroxybutyric acid and sodium R-3-hydroxybutyrate; the stability of 3-hydroxybutyric acid · sodium 3-hydroxybutyrate complex is very high, which is significantly higher than that of 3-hydroxybutyric acid, and mixture of 3-hydroxybutyric acid and sodium 3-hydroxybutyrate; the stability of S-3-hydroxybutyric acid · sodium S-3-hydroxybutyrate complex is very high, which is significantly higher than that of S-3-hydroxybutyric acid, and mixture of S-3-hydroxybutyric acid and sodium S-3-hydroxybutyrate_{∘}

The invention solves the problems of strong acidity, intestinal side effects, high hygroscopicity and poor stability of BHB acid, and simultaneously solves the electrolyte imbalance caused by high salt load of BHB salt. The complex shows better comprehensive effect than acid or salt alone or simple physically mixed components, and has appropriate hygroscopicity and stability, so that it is particularly suitable for the preparation of solid preparations. Secondly, at a proper dosage, the ketogenic effect of the complex of the invention is substantially equivalent to that of BHB acid and BHB salt, and at a medium dosage, the ketogenic effect is better than that of BHB salt. In addition, the complex prepared by the invention has high purity, uniform particle size distribution, good fluidity, low adhesiveness and good bioavailability; and the preparation process is controllable, at low cost, and environmentally friendly.

The above are only the preferred embodiments of the present invention, and are not intended to limit the present invention. Those skilled in the art can make many changes, modifications, substitutions and variations on these embodiments without departing from the principles and purposes of the invention, and the scope of the invention is defined by the claims and their equivalents.

## Claims

1. A complex comprising 3-hydroxybutyric acid and sodium 3-hydroxybutyrate.

2. The complex of claim 1, wherein anions in the complex structure include 3-hydroxybutyrate anions, and cations include sodium ions and hydrogen ions.

3. The complex of claim 1 or 2, wherein the ratio of 3-hydroxybutyric acid to sodium 3-hydroxybutyrate is 1:10 to 10:1.

4. The complex of any one of claims 1 to 3, wherein the complex contains not less than 50% of R configuration, not more than 50% of S configuration; or more than 50% of S configuration, less than 50% of R configuration.

5. The complex of any one of claims 1 to 4, wherein the complex is 3-hydroxybutyric acid · sodium 3-hydroxybutyrate.

6. The complex of any one of claims 1 to 5, wherein the complex has the following structure:

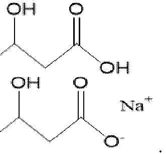

7. The complex of any one of claims 1 to 6, wherein the complex is R-3-hydroxybutyric acid · sodium R-3-hydroxybutyrate and/or S-3-hydroxybutyric acid · sodium S-3-hydroxybutyrate.

8. The complex of any one of claims 1 to 6, wherein the complex contains not less than 50% of R-3-hydroxybutyric acid · sodium R-3-hydroxybutyrate, not more than 50% of S-3-hydroxybutyric acid · sodium S-3-hydroxybutyrate; or more than 50% of S-3-hydroxybutyric acid · sodium S-3-hydroxybutyrate, less than 50% of R-3-hydroxybutyric acid · sodium R-3-hydroxybutyrate_{∘}

9. The complex of claim 7 or 8, wherein the complex has the following structures:

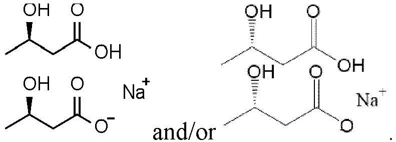

10. The complex of any one of claims 1 to 9, wherein the complex is in crystalline form.

11. The complex of any one of claims 1 to 10, wherein the X-ray powder diffraction pattern of the complex further comprises peaks at diffraction angles (2θ) of 9.8°±0.2°, 10.8°±0.2°, 18.9±0.2°, and 31.1°±0.2°.

12. The complex of claim 11, wherein the X-ray powder diffraction pattern of the complex further comprises one or more peaks at diffraction angles (2θ) of 13.4°±0.2°, 21.6°±0.2°, 32.7°±0.2°.

13. The complex of claim 11 or 12, wherein the X-ray powder diffraction pattern of the complex further comprises one or more peaks at diffraction angles (2θ) of 15.1°±0.2°, 23.0°±0.2°, 31.9°±0.2°.

14. The complex of any one of claims 1 to 10, wherein the X-ray powder diffraction pattern of the complex is shown in Figure 1A, Figure 1B or Figure 1C.

15. The complex of any one of claims 1 to 10, wherein the infrared spectrum of the complex has the following absorption bands, expressed in reciprocal wavelengths (cm⁻¹) (±2 cm⁻¹): 1715, 1452, 1414, 1368, 1306, 1217, 1148, 1094, 1057, 980, 853, 710, 573, 467.

16. The complex of any one of claims 1 to 10, wherein the X-ray powder diffraction pattern of the complex further comprises peaks at diffraction angles (2θ) of 6.9°±0.2°, 10.7°±0.2°, 18.1°±0.2°, 22.3°±0.2°, 23.0±0.2°, 26.8°±0.2°, and 31.2°±0.2°.

17. The complex of claim 16, wherein the X-ray powder diffraction pattern of the complex further comprises one or more peaks at diffraction angles (2θ) of 17.2°±0.2°, 21.6°±0.2°, 25.8°±0.2°.

18. The complex of any one of claims 1 to 10, wherein the X-ray powder diffraction pattern of the complex further comprises peaks at diffraction angles (2θ) of 7.7°±0.2°, 10.7°±0.2°, 13.2°±0.2°, 18.7±0.2°, 21.4°±0.2°, 31.4°±0.2°, and 32.6°±0.2°.

19. The complex of claim 18, wherein the X-ray powder diffraction pattern of the complex further comprises one or more peaks at diffraction angles (2θ) of 15.0°±0.2°, 23.9°±0.2°, 34.3°±0.2°.

20. The complex of any one of claims 1 to 19, wherein the complex is prepared as food, beverage, supplement or pharmaceutical preparation.

21. A 3-hydroxybutyric acid · sodium 3-hydroxybutyrate complex, wherein the complex is obtained by the following method:
(1) obtaining a substance A by one of the following methods: mixing 3-hydroxybutyric acid and sodium 3-hydroxybutyrate; or adding 3-hydroxybutyric acid into an aqueous solution of an alkaline Na compound, stirring, removing water, and evaporating to near dryness; or reacting alkyl 3-hydroxybutyrate with water under heating in the presence of a catalyst, cooling and filtering, adding an aqueous solution of an alkaline Na compound to the filtrate, distilling under reduced pressure to remove water, and evaporating to near dryness;
(2) adding one or more solvents selected from the following into the substance A obtained in step (1): water, THF, DMF, DMSO, DMAC, alcohol, halogenated hydrocarbon, ketone, ester, stirring and cooling to precipitate a solid;
(3) filtering out the solid and drying to obtain the complex.

22. The complex of claim 21, wherein the complex is R-3-hydroxybutyric acid · sodium R-3-hydroxybutyrate and/or S-3-hydroxybutyric acid · sodium S-3-hydroxybutyrate.

23. The complex of claim 21 or 22, wherein the complex is in crystalline form.

24. A method for preparing the complex of any one of claims 1 to 10, wherein the method includes the following steps:
(1) a substance A is obtained by one of the following methods: mixing 3-hydroxybutyric acid and sodium 3-hydroxybutyrate; or adding 3-hydroxybutyric acid into an aqueous solution of an alkaline Na compound, stirring, removing water, and evaporating to near dryness; or reacting alkyl 3-hydroxybutyrate with water under heating in the presence of a catalyst, cooling and filtering, adding an aqueous solution of an alkaline Na compound to the filtrate, distilling under reduced pressure to remove water, and evaporating to near dryness;
(2) adding one or more solvents selected from the following into the substance A obtained in step (1): water, THF, DMF, DMSO, DMAC, alcohol, halogenated hydrocarbon, ketone, ester, stirring and cooling to precipitate a solid;
(3) filtering out the solid and drying to obtain the complex.

25. The method of claim 24, wherein in step (2), the alcohol is methanol, ethanol, isopropanol, n-butanol; the halogenated hydrocarbon is chlorobenzene, dichlorobenzene, dichloromethane; the ketone is acetone, methyl butanone, methyl isobutyl ketone; the ester is ethyl acetate, isopropyl acetate, n-butyl acetate, isobutyl acetate.

26. The method of claim 24 or 25, wherein in step (1), the alkaline Na compound is NaOH, Na₂CO₃, NaHCO₃, NaOMe, NaOAc or NaOCHO; the alkyl 3-hydroxybutyrate is methyl 3-hydroxybutyrate, ethyl 3-hydroxybutyrate, propyl 3-hydroxybutyrate, butyl 3-hydroxybutyrate.

27. A composition comprising an effective amount of the complex of any one of claims 1 to 23, and a pharmaceutically acceptable carrier.

28. The composition of claim 27, wherein the composition is used as a ketogenic substance.

29. The composition of claim 27 or 28, wherein the composition is prepared as food, beverage, supplement or pharmaceutical preparation.

30. Use of the complex of any one of claims 1 to 23, wherein the complex is used for preparing a ketogenic substance for increasing or maintaining blood ketone level of a subject.

31. The use of claim 30, wherein the ketogenic substance is nutritional supplement, energy therapy, medical treatment or strength and/or endurance exercise supplement.

32. Use of a composition in preparing a ketogenic substance for increasing or maintaining blood ketone level of a subject, wherein the composition comprises the complex of any one of claims 1 to 23, and a pharmaceutically acceptable carrier.

33. The use of claim 32, wherein the ketogenic substance is nutritional supplement, energy therapy, medical treatment or strength and/or endurance exercise supplement.
